(19) Europäisches Patentamt
European Patent Office
Office européen des brevets

(11) **EP 2 078 976 A1**

(12) **EUROPEAN PATENT APPLICATION**

(43) Date of publication:
**15.07.2009 Bulletin 2009/29**

(51) Int Cl.:
***G02B 23/24*** *(2006.01)* ***A61B 1/005*** *(2006.01)*

(21) Application number: **09000144.7**

(22) Date of filing: **08.01.2009**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **08.01.2008 JP 2008001201**

(71) Applicant: **Olympus Medical Systems Corporation Tokyo 151-0072 (JP)**

(72) Inventor: **Akiyama, Daisuke Hachioji-shi Tokyo 192-8512 (JP)**

(74) Representative: **von Hellfeld, Axel Wuesthoff & Wuesthoff Patent- und Rechtsanwälte Schweigerstrasse 2 81541 München (DE)**

(54) **Objective optical system for endoscopes and endoscope system using the same**

(57) An objective optical system for endoscopes is provided for the purpose of carrying out a fluorescence observation. At least two excitation light cutoff filters in which the transmittance of excitation light in its wavelength band is 0.1 % or less are arranged and an optical element with power is interposed between arbitrary two of the excitation light cutoff filters. Whereby, the leakage of the excitation light caused by oblique incidence on the excitation light cutoff filter can be effectively prevented and even the feeble fluorescent light can be observed without flare with respect to the excitation light.

EP 2 078 976 A1

## Description

## BACKGROUND OF THE INVENTION

1. Field of the Invention

This invention relates to an objective optical system for endoscopes, notably an objective optical system for fluorescence endoscopes, and an endoscope system using this objective optical system.

2. Description of Related Art

In a fluorescence observation, for excitation light the intensity is roughly 1000 times as high as for fluorescent light, and hence even when part of the excitation light reaches an imaging surface, an S/N ratio is impaired. Consequently, various provisions are made for preventing the penetration of the excitation light on the imaging surface to improve the S/N ratio.

As one of the provisions, for example, Japanese Patent Kokai No. 2001-128927 discloses the structure that auto-fluorescence produced from a living tissue by the irradiation of excitation light is detected through a plurality of excitation light cutoff filters and a first-stage excitation cutoff filter, of the plurality of excitation cutoff filters, is taken as a filter in which fluorescent light is not produced by the irradiation of the excitation light. Further, Japanese Patent Kokai No. 2004-344230 discloses the structure that an excitation light cutoff filter reducing the transmittance of excitation light to 0.1 % or less is provided in an objective optical system in which an image of an object is formed and a light passage preventing means is provided to block excitation light passing through a gap between an outer surface portion of the excitation cutoff filter and an inner surface portion of a holding frame for holding the excitation light cutoff filter.

In the structure such as proposed by Kokai No. 2004-344230, excitation light from inside the visual field of the objective optical system, namely excitation light traveling nearly parallel to the optical axis of the objective optical system, is blocked by the excitation light cutoff filter and fails to reach an image sensor. However, when a thin film filter (an optical thin film filter) suitable for the excitation light cutoff filter is adopted, it has the property of changing its characteristic with respect to light of oblique incidence to transmit the light, and thus is transparent to excitation light from outside the visual field of the objective optical system which is reflected and/or scattered by the folding frame and a lens side surface to increase the angle of incidence on the excitation light cutoff filter. This transmitted excitation light is reflected directly or by the holding frame to reach the image sensor and thereby becomes flare. The amount of auto-fluorescence observed by a CCD as the image sensor is no more than 0.1 % of that of the excitation light, and hence even though the transmitted excitation light is multiple scattered light, it has the intensity enough to constitute an obstacle to the fluorescence observation.

In particular, since the objective optical system for endoscopes is constructed so that the holding frame is closely placed in a short optical path because of the need of a compact design, reflected and/or scattered light caused by the holding frame and the lens side surface is liable to return into the optical path. In a conventional observation, the light is completely attenuated and thus the light reaching the CCD has no problem. In the fluorescence observation, however, light obliquely incident on the filter and reflected and/or scattered is recognized as flare to cause a problem.

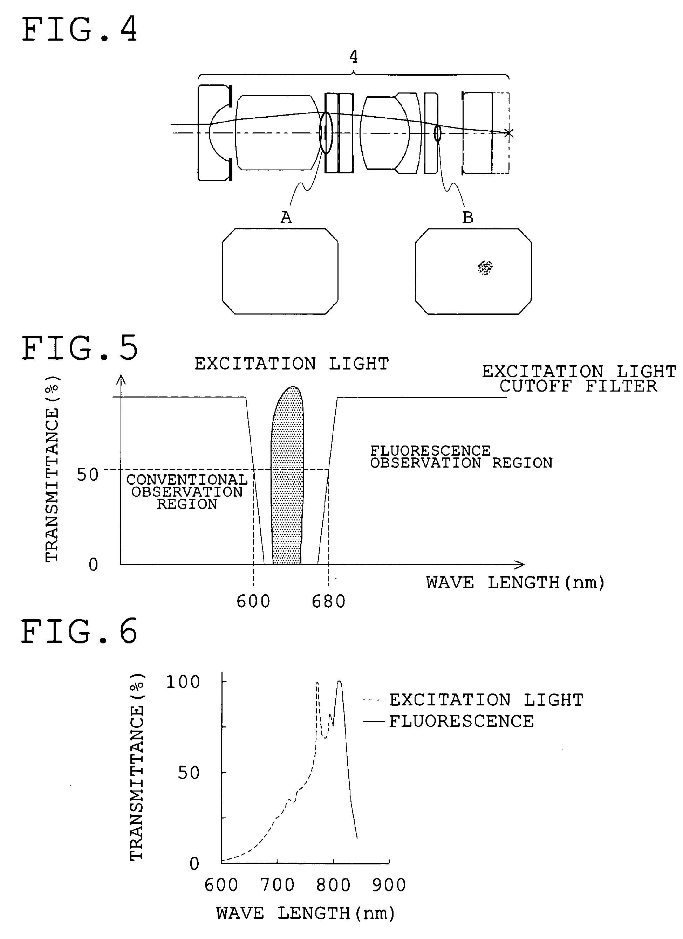

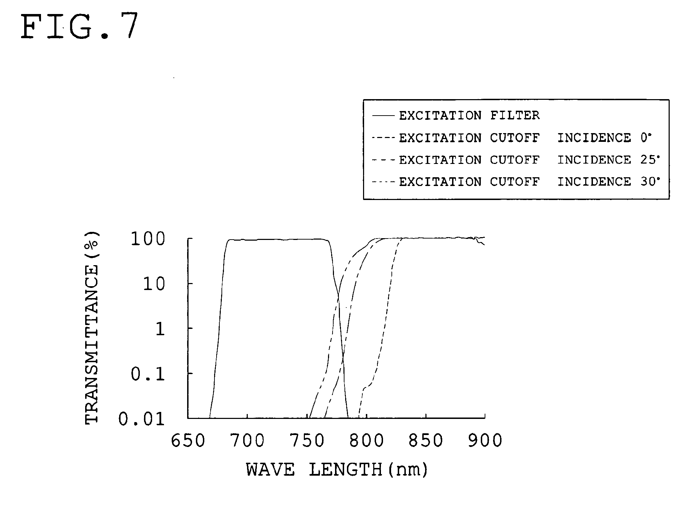

It is generally known that, in order to completely block the excitation light by the excitation light cutoff filter, it is good practice to set the transmittance of the excitation light in its wavelength band to at most 0.1 %. In many of fluorescent substances, an absorption wavelength band is close to an excitation wavelength band (refer to Fig. 6), and in order to transmit fluorescent light while completely blocking the excitation light, a steep transmittance characteristic is required for the filter. Therefore, the thin film filter (the optical thin film filter) is often used for the excitation light cutoff filter. On the other hand, the thin film filter (the optical thin film filter) has the characteristic of the angle of incidence and acquires the property of shifting the characteristic to the short-wavelength side as the angle of incidence of light increases (refer to Fig. 7). The amount of this shift increases with increasing wavelength of light and thus when fluorescent light in a red to near-infrared wavelength region is taken as an observation target, its influence becomes more pronounced.

Generally, in the thin film filter based on a dielectric multilayer film, when a reference wavelength that an interference effect is maximized at an angle of incidence of 0° is denoted by $\lambda_0$, the reference wavelength $\lambda_0$ and an reference wavelength $\lambda$ at an angle of incidence $\theta$ have the following relation:

$$\lambda \propto \lambda_0 \cdot \cos \theta$$

This is a formula showing that as the angle of incidence of light increases, its characteristic is shifted to the short-wavelength side, but it also shows that as the reference wavelength increases, the amount of shift increases. For example, the amount of shift of the characteristic in the case where a filter with a reference wavelength of 500 nm is used in order to observe green fluorescent light is nearly 1.5 times that of the characteristic in the case where a filter with a reference wavelength of 750 nm is used in order to observe near-infrared fluorescent light. When fluorescent light in the red to near-infrared wavelength region is taken as an observation target, the influence of flare becomes more pronounced.

## SUMMARY OF THE INVENTION

It is, therefore, an object of the present invention to provide an objective optical system for endoscopes and an endoscope system in which the leakage of the excitation light caused by oblique incidence on the excitation light cutoff filter can be effectively prevented and even the feeble fluorescent light can be observed without flare with respect to the excitation light.

In order to achieve the above object, the objective optical system for endoscopes according to the present invention is provided for the purpose of carrying out a fluorescence observation, and in this case, at least two excitation light cutoff filters in which the transmittance of excitation light in its wavelength band is 0.1 % or less are arranged and an optical element with power is interposed between arbitrary two of the excitation light cutoff filters. Owing to this arrangement, the angle of a light ray is changed by the optical element with respect to the excitation light incident on one excitation light cutoff filter at a large angle and transmitted, and thus the light can be blocked by the other excitation light cutoff filter.

According to the present invention, each of the excitation light cutoff filters has a transmittance in a red to near-infrared light region. Since the influence of the characteristic of the angle of incidence increases with increasing wavelength of light, a filter having a transmission range in the red to near-infrared light region is used for the excitation light cutoff filter and thereby the effect of the present invention can be exerted more prominently.

According to the present invention, the excitation light cutoff filter is such that a wavelength in a 50 percent transmittance of a ray at an angle of incidence of 0° is 680 nm or more. By this arrangement, a near-infrared fluorescence observation can be carried out as the conventional observation using light with wavelengths of 380-600 nm. When the wavelength width of the excitation light and the wavelength shift by the oblique incidence are taken into account, it is desirable that the wavelength in the 50 percent transmittance of the ray at the angle of incidence of 0° is 680 nm or more at a short-wavelength cutoff end of the transmittance spectrum of the excitation light cutoff filter (refer to Fig. 5).

According to the present invention, the optical element interposed between the two excitation light cutoff filters has a positive power. By this arrangement, light obliquely incident on the excitation light cutoff filters can be further curved toward the optical axis.

According to the present invention, at least one of the excitation light cutoff filters is located proximate to the pupil position of the optical system. By this arrangement, the influence of a partial leakage of the excitation light attributable to the problem of the fabrication of the excitation light cutoff filter on the image can be favorably controlled. Since a beam diameter is large in the proximity of the pupil, the extent of the leakage of the excitation light is relatively small and the S/N ratio is improved.

According to the present invention, the objective optical system for endoscopes comprises, in order from the object side, a first lens unit with negative power, a second lens unit with positive power, and a third lens unit with positive power, and at least one excitation light cutoff filter is located behind the third lens unit.

By this arrangement, the objective optical system that is compact and has a high S/N ratio can be provided.

The endoscope system according to the present invention has an excitation filter transmitting excitation light in an illumination optical path between a light source lamp and an observation object so that the object irradiated with the excitation light is observed through an objective optical system including the excitation light cutoff filter. In this case, the endoscope system has the objective optical system in which at a wavelength in a 0.1 percent transmittance of a ray at an angle of incidence of 0° on the excitation filter, the transmittance of a ray at an angle of incidence of 25° on the excitation light cutoff filter is 0.1 % or more. By this arrangement, the transmission range of the excitation filter can be brought close to that of the excitation light cutoff filter in accordance with the absorption wavelength and the fluorescence wavelength of the fluorescent substance, and a more effective fluorescence observation can be carried out.

According to the present invention, the objective optical system for endoscopes and the endoscope system can be provided in which the leakage of the excitation light by the oblique incidence on the excitation light cutoff filter can be effectively prevented with increasing observation wavelength, and even the feeble fluorescent light can be observed without flare with respect to the excitation light.

These and other features and advantages of the present invention will become apparent from the following detailed description of the preferred embodiment when taken in conjunction with the accompanying drawings.

## BRIEF DESCRIPTION OF THE DRAWINGS

Fig. 1 is a schematic view showing the entire structure of one example of the endoscope system having the objective optical system for endoscopes according to the present invention.
Fig. 2 is a sectional view showing an optical arrangement, developed along the optical axis, of one embodiment of the objective optical system for endoscopes according to the present invention.
Fig. 3 is a partially enlarged sectional view showing a positional relationship between an aperture stop and an excitation light cutoff filter in the objective optical system for endoscopes of Fig. 2.
Fig. 4 is an explanatory view showing beam diameters on a filter surface close to the aperture stop and on a filter surface at another place and a pinhole image on the imaging surface where the placement position of a first excitation light cutoff filter is changed, in the objective optical system for endoscopes of Fig. 2.

Fig. 5 is an explanatory view showing the transmittance spectrum of the excitation light cutoff filter.

Fig. 6 is a transmittance characteristic diagram showing the relationship between the excitation light wavelength and the absorption wavelength of the fluorescent substance.

Fig. 7 is a transmittance characteristic diagram showing the relationship between a transmittance characteristic of the excitation filter and a change of a transmittance characteristic of the excitation light cutoff filter due to the difference of the angle of incidence of light, in the present invention.

## DETAILED DESCRIPTION OF THE PREFERRED EMBODIMENT

In accordance with the embodiment shown in the drawings, the present invention will be explained below.

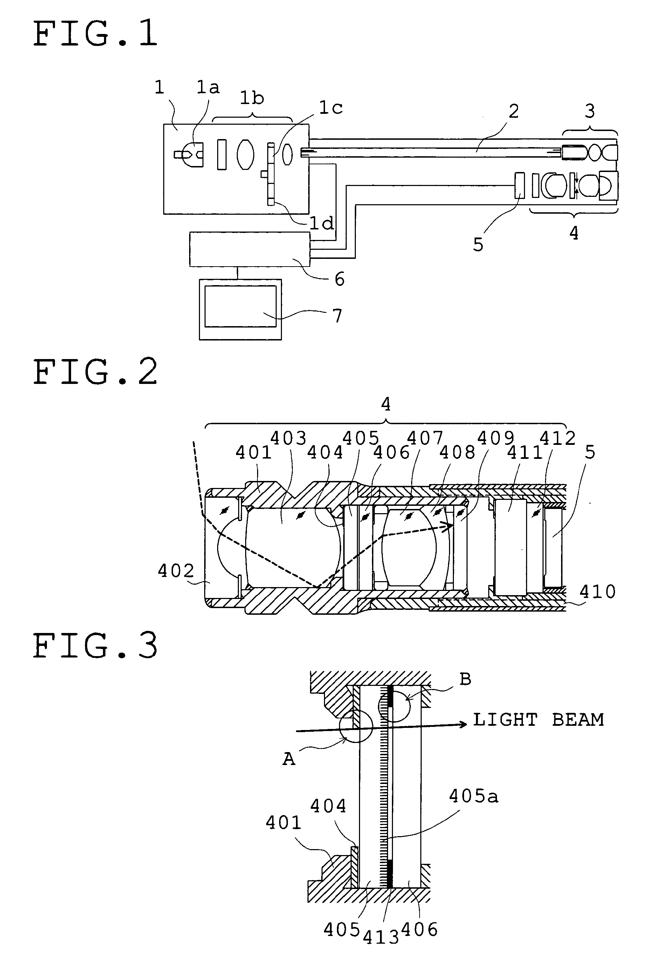

Fig. 1 is a schematic view showing the entire structure of one example of the endoscope system having the objective optical system for endoscopes according to the present invention. Fig. 2 is a sectional view showing an optical arrangement, developed along the optical axis, of one embodiment of the objective optical system for endoscopes according to the present invention. Fig. 3 is a partially enlarged sectional view showing a positional relationship between an aperture stop and an excitation light cutoff filter in the objective optical system for endoscopes of Fig. 2.

In Fig. 1, reference numeral 1 represents a light source device provided with a light source lamp 1a, a condenser optical system 1b, a rotary filter plate 1d including an excitation filter 1c, etc.; 2, a light guide introducing excitation light emitted from the light source device 1 into an illumination optical system 3; 4, an objective optical system, described later, according to the present invention; 5, an image sensor, such as a high-sensitivity CCD, receiving an object image formed by the objective optical system 4; 6, an image processor imaging an image signal obtained by the image sensor 5; and 7, a monitor displaying the object image. The light guide 2, the illumination optical system 3, the objective optical system 4, and the image sensor 5, as well known, are incorporated in a rigid tube constituting the distal end of the endoscope.

Fig. 2 shows a detailed arrangement of the objective optical system 4. In this figure, reference numeral 401 denotes a lens frame, and in the lens frame 401, a plano-concave lens 402, a biconvex lens 403, an aperture stop 404, a first excitation light cutoff filter 405, an optical filter 406, a biconvex lens 407, a negative meniscus lens 408 with a concave surface facing the object side, and a second excitation light cutoff filter 409 are arranged, in this order from the object side, through a holding ring at preset intervals. Also, the image sensor 5 is held in a holding tube 410 into which the lens frame 401 is fitted and a cover glass 411 is placed on the front side (on the object side) of the image sensor 5. Here, the plano-concave lens 402 constitutes a first lens unit with negative power, the biconvex lens 403 constitutes a second lens unit with positive power, and the biconvex lens 407 and the negative meniscus lens 408 constitute a third lens unit with positive power. Also, the first and second excitation light cutoff filters 405 and 409 each have a transmission range in the red to near-infrared light region and the transmittance of one of the filters is selected so that the transmittance of a ray at an angle of incidence of 0° in the wavelength band of the excitation light is 0.1 % or less, the transmittance of a ray at angle of incidence of 25° is 0.1 % or more, and the wavelength in a 50 percent transmittance of a ray at an angle of incidence of 0° is 680 nm or more.

Since the objective optical system for endoscopes according to the present invention is constructed as mentioned above, a ray of light (excitation light), as indicated by a broken line in Fig. 2, which is incident on the objective lens 402 at a considerable angle with the optical axis to enter the objective optical system 4 and is reflected by the peripheral surface of the biconvex lens 403 to obliquely enter the first excitation light cutoff filter 405, is bent toward the optical axis by the biconvex lens 407 and enters the second excitation light cutoff filter 409. Hence, the excitation light is efficiently blocked and there is no light traveling through the second excitation light cutoff filter 409 and reflected and scattered by the inner wall surface of the holding tube 410. Consequently, the S/N ratio can be materially improved and even the feeble fluorescent light can be easily observed without flare with respect to the excitation light.

As will be obvious from this description, the third lens unit is not limited to the biconvex lens 407 and a prism can be used instead of the biconvex lens. In short, it is only necessary to preferably use an optical element with positive power.

Fig. 3 is a detailed view showing the positional relationship with the aperture stop 404, the first excitation light cutoff filter 405, and optical filter 406. As will be seen from this figure, the first excitation light cutoff filter 405 is located so that its surface 405a that chiefly exerts the function of blocking the excitation light lies on the side where it does not come in contact with the aperture stop 404, and a blocking member 413 that is in contact with the surface 405a and determines the aperture of the excitation light cutoff filter 405 is located to lie outside a light beam governed by the aperture stop 404.

Therefore, even though the aperture stop 404 and the first excitation light cutoff filter 405 are rubbed together, for example, by vibration and a part indicated by an arrow A is damaged, it is avoidable that the leakage of the excitation light is caused by the damaged part to impair an excitation light cutoff function. In addition, even though a part indicated by an arrow B with which the excitation light cutoff surface 405a and the blocking member 413 come in contact is damaged, the observation is not affected.

Also, in the excitation light cutoff filter of this type, the occurrence of a pinhole cannot be entirely avoided for reasons of fabrication. However, as seen from Fig. 3, the first excitation light cutoff filter 405 is located close to the pupil position of the objective optical system and thus as shown in Fig. 4, the leakage of the excitation light through the pinhole is moderated. Furthermore, since the second excitation light cutoff filter 409 is located, with the lens unit of a positive focal length including the lenses 407 and 408 between the first and second excitation light cutoff filters, no image is formed on the imaging surface by the leakage of the excitation light through the pinhole and an obstruction to the observation is not offered. In Fig. 4, reference symbol A denotes a pinhole image on the imaging surface where the excitation light cutoff filter having the pinhole is located close to the aperture stop 404 and B denotes a pinhole image on the imaging surface where the excitation light cutoff filter is not located close to the aperture stop 404 and is located at another position only. As is obvious from this figure, since the beam diameter is largest in the proximity of the aperture stop 404, a relative size of the pinhole to the light beam becomes small and the S/N ratio is improved.

Also, although the two excitation light cutoff filters are used in the embodiment, the present invention is not limited to the number of these filers, and three or more excitation light cutoff filters may be used if need arises. In any case, it is necessary that the third lens unit is interposed between arbitrary two of the excitation light cutoff filters.

According to the present invention, as will be clear from the above description, the objective optical system for endoscopes and the endoscope system which are favorable for the fluorescence observation in the red to near-infrared wavelength region can be provided.

**Claims**

1. An objective optical system for endoscopes provided for a purpose of carrying out a fluorescence observation, wherein at least two excitation light cutoff filters in which a transmittance of excitation light in a wavelength band thereof is 0.1 % or less are arranged and an optical element with power is interposed between arbitrary two of the excitation light cutoff filters.

2. An objective optical system for endoscopes according to claim 1, wherein each of the excitation light cutoff filters has a transmittance in a red to near-infrared light region.

3. An objective optical system for endoscopes according to claim 2, wherein a wavelength in a 50 percent transmittance of a ray at an angle of incidence of 0° is 680 nm or more.

4. An objective optical system for endoscopes according to any one of claims 1-3, wherein the optical element interposed between the two excitation light cutoff filters has a positive power.

5. An objective optical system for endoscopes according to any one of claims 1-4, wherein at least one of the excitation light cutoff filters is located proximate to a pupil position of the optical system.

6. An objective optical system for endoscopes according to claim 5, comprising, in order from an object side, a first lens unit with negative power, a second lens unit with positive power, and a third lens unit with positive power, wherein at least one excitation light cutoff filter is located behind the third lens unit.

7. An endoscope system having an excitation filter transmitting excitation light in an illumination optical path between a light source lamp and an object so that an observation object irradiated with the excitation light is observed through an objective optical system including the excitation light cutoff filter, wherein the endoscope system has the objective optical system according to any one of claims 1-6 in which at a wavelength in a 0.1 percent transmittance of a ray at an angle of incidence of 0° on the excitation filter, a transmittance of a ray at an angle of incidence of 25° on the excitation light cutoff filter is 0.1 % or more.

# FIG.1

# FIG.2

# FIG.3

# FIG.4

# FIG.5

EXCITATION LIGHT

EXCITATION LIGHT
CUTOFF FILTER

FLUORESCENCE
OBSERVATION REGION

CONVENTIONAL
OBSERVATION
REGION

WAVE LENGTH (nm)

# FIG.6

--- EXCITATION LIGHT
— FLUORESCENCE

WAVE LENGTH (nm)

7

# FIG.7

| | |
|---|---|
| —— | EXCITATION FILTER |
| ---- | EXCITATION CUTOFF  INCIDENCE 0° |
| --- | EXCITATION CUTOFF  INCIDENCE 25° |
| ---- | EXCITATION CUTOFF  INCIDENCE 30° |

**EUROPEAN SEARCH REPORT**

Application Number

EP 09 00 0144

Europäisches
Patentamt

European
Patent Office

Office européen
des brevets

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/233538 A1 (SUGA TAKESHI [JP]) 25 November 2004 (2004-11-25) * figure 6 * * paragraph [0005] * * paragraph [0014] - paragraph [0018] * * paragraph [0049] * * paragraph [0119] * * paragraph [0167] - paragraph [0173] * ----- | 1,4-6 | INV. G02B23/24 A61B1/005 |
| X | US 2005/027166 A1 (MATSUMOTO SHINYA [JP] ET AL) 3 February 2005 (2005-02-03) * figures 1,3,5,21(a)-21(c) * * abstract * * paragraph [0014] * * paragraph [0119] * * paragraph [0127] - paragraph [0128] * * paragraph [0167] - paragraph [0173] * ----- | 1-5,7 | |
| A | US 2003/229270 A1 (SUZUKI TAKAYUKI [JP] ET AL) 11 December 2003 (2003-12-11) * paragraph [0126]; figures 25(a)-(b) * ----- | 7 | |

TECHNICAL FIELDS SEARCHED (IPC)

G02B
A61B

The present search report has been drawn up for all claims

| Place of search | Date of completion of the search | Examiner |
|---|---|---|
| The Hague | 19 May 2009 | Vanderperren, Yves |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

EP 2 078 976 A1

**ANNEX TO THE EUROPEAN SEARCH REPORT
ON EUROPEAN PATENT APPLICATION NO.**

EP 09 00 0144

This annex lists the patent family members relating to the patent documents cited in the above-mentioned European search report.
The members are as contained in the European Patent Office EDP file on
The European Patent Office is in no way liable for these particulars which are merely given for the purpose of information.

19-05-2009

| Patent document cited in search report | | | Publication date | Patent family member(s) | | Publication date |
|---|---|---|---|---|---|---|
| US 2004233538 | A1 | | 25-11-2004 | JP | 2004344230 A | 09-12-2004 |
| US 2005027166 | A1 | | 03-02-2005 | NONE | | |
| US 2003229270 | A1 | | 11-12-2003 | JP | 4054222 B2 | 27-02-2008 |
| | | | | JP | 2004008412 A | 15-01-2004 |

EPO FORM P0459

For more details about this annex : see Official Journal of the European Patent Office, No. 12/82

**EP 2 078 976 A1**

**Patent documents cited in the description**

- JP 2001128927 A

- JP 2004344230 A